# EUROPEAN PATENT SPECIFICATION

(11) **EP 2 350 348 B1**
(45) Date of publication and mention of the grant of the patent: **14.06.2017**
(21) Application number: 09818847.7
(22) Date of filing: 01.10.2009
(51) Int. Cl.: C25B 1/02, H01M 8/16, H01M 8/06, C12N 9/02, C12P 3/00, H01M 8/0656

(54) **METHOD AND SYSTEM FOR PRODUCING HYDROGEN, AND ELECTRICITY GENERATION SYSTEM**
VERFAHREN UND SYSTEM ZUR HERSTELLUNG VON WASSERSTOFF UND SYSTEN ZUR STROMERZEUGUNG
PROCÉDÉ ET SYSTÈME DE PRODUCTION D'HYDROGÈNE ET SYSTÈME DE PRODUCTION D'ÉLECTRICITÉ

(30) Priority: 08.10.2008 FI 20085946
(43) Date of publication of application: 03.08.2011
(73) Proprietor: Aalto University Foundation, 00076 Aalto (FI)
(72) Inventor: HALME, Aarne, FI-02180 Espoo (FI); RANTA, Anja, FI-02120 Espoo (FI)
(74) Representative: Kolster Oy Ab
(86) International application number: PCT/FI2009/050793
(87) International publication number: WO 2010/040897

(56) References cited:
- EP-A1- 1 939 961
- EP-A2- 1 376 729
- JP-A- 2000 331 702
- US-A1- 2002 127 440
- US-A1- 2005 095 466
- US-A1- 2006 011 491
- US-A1- 2007 108 065
- US-A1- 2007 184 309
- YOU-KWAN OH ET AL: 'Bioelectrocatalytic hydrogen production using Thiocapsa roseopersicina hydrogenase in two-compartment fuel cells' INTERNATIONAL JOURNAL OF HYDROGEN ENERGY vol. 33, no. 19, October 2008, pages 5218 - 5223, XP008136867
- BULLEN R.A. ET AL: 'Biofuel cells and their development' BIOSENSORS AND BIOELECTRONICS vol. 21, no. 11, May 2006, pages 2015 - 2045, XP024961489
- CRACKNELL J.A. ET AL: 'Enzymes as working or inspirational electrocatalysts for fuel cells and electrolysis' CHEMICAL REVIEWS vol. 108, no. 7, July 2008, pages 2439 - 2461, XP008136868

## Description

### BACKGROUND OF THE INVENTION

The invention relates to a method and a system for producing hydrogen. In particular, the invention relates to electrolytic hydrogen production from an organic substrate by using an enzyme catalyst. The produced hydrogen is conveyed to a fuel cell of a PEM type, in which the chemical energy of the hydrogen fuel is converted into electric energy. The invention also relates to a system which comprises a unit for producing hydrogen, a PEM fuel cell using hydrogen as fuel, intended for generating electricity, and an electronic current control unit.

US publication 2007/0184309 A1 discloses a method for producing hydrogen in situ by means of a photobiofuel cell from organic fuels. A photobiofuel cell comprises a light-sensitive photoanode in an aqueous medium, the medium comprising NADH coenzyme, a fuel and an enzyme to maintain NADH levels. The fuel decomposes by means of an enzyme in an anode compartment, whereby electrons, protons and by-products are generated. The electrons are transferred, by means of an NAH⁺/NADH redox pair, to the anode and from there further to a cathode, generating an electric current. The fuel cell requires a light source for illuminating the photoanode and providing the necessary electrode potential. Producing hydrogen takes place at the cathode by reducing the protons having travelled to it, while a hydrogenase enzyme catalyzes.

EP publication 1376729 A2 discloses a biocatalytic direct alcohol fuel cell and the use thereof for producing electric power. The fuel cell is applicable to an energy source for low-power electronic devices. The fuel cell comprises an anode chamber and a cathode chamber, and an ion exchange membrane between the chambers. The anode chamber contains fuel and enzyme for oxidation of fuel. Particularly lower alcohols are mentioned as the fuel. The cathode chamber contains a chemical catalyst, an enzyme or a combination thereof for reduction of oxygen.

JP publication 2003308869 discloses a fuel cell in which methanol is used as the fuel for generating electric power. The fuel cell comprises an electrolytic unit in which the methanol is decomposed to produce hydrogen and, integrated therewith, a PEM fuel cell unit using the hydrogen as the fuel. By means of the fuel cell, electricity can be generated intermittently.

DE utility model application 29823321 U1 describes an electrolytic fuel cell system comprising an oxygen electrode and a hydrogen electrode as well as a solid electrolytic membrane between them. The system also comprises catalyst, whereby a mixed catalyst is used at the oxygen electrode, the mixed catalyst comprising oxygen-reducing components, such as platinum, and hydrogen-oxidizing components, such as iridium, rhodium or osmium. This cell system operates at low overvoltages, and its operation may be varied quickly between the electrolytic and fuel cell compartments.

The NASA publication Making Hydrogen by Electrolysis of Methanol, NASA Jet Propulsion Laboratory, Pasadena, California, 1 June 2002 (NASA Tech Briefs, 2008), studies electrolysis of methanol into carbon dioxide and hydrogen which may be used in fuel cells. Reactions taking place at both an anode and a cathode are catalyzed by a metal catalyst, such as a platinum catalyst. It becomes apparent from the study that less energy is required for electrolysis of methanol than for electrolysis of water.

US 2008/0152967 A1 discloses a method for producing hydrogen from sewage sludge which is decomposed electrolytically in an anaerobic digester. Digestion is performed by means of microbes present in the sludge.

US 2007/0184309 A1 discloses a method for producing hydrogen by using a photobiofuel cell. Hydrogen is produced from organic fuels by using an NADH coenzyme.

US 2007/018065 A1 discloses a method for producing hydrogen wherein water is treated electrolytically.

One problem in the arrangements described above is the use of noble metal catalysts in the electrolytic fuel cell systems. Such catalysts are known to be expensive and their availability is limited. On the other hand, the power density of the liquid fuel cell of EP publication 1376729 A2, for example, in generation of electricity is not of the same order of magnitude as in the system according to the invention but significantly lower than in this system where the organic substrate used as the primary fuel, such as methanol, is first converted into hydrogen and only then into electricity in a gas fuel cell. In a gas fuel cell, the same electric power is generated in a significantly smaller volume than in a liquid fuel cell.

### BRIEF DESCRIPTION OF THE INVENTION

Decomposition of water into hydrogen and oxygen by means of electrolysis is a method generally known. It enables production of hydrogen of a high purity level and, in addition, production of oxygen by means of simple equipment. A drawback of the method is the great electricity consumption required for the electrolysis of water. Normally, water begins to decompose when the electrode voltage between an anode and a cathode is about 1.4 V to 1.8 V. Production of hydrogen is directly proportional to the current generated between the electrodes.

Now, a novel method has been invented in which hydrogen is produced by decomposing an aqueous solution of an alcohol instead of water by utilizing an enzyme catalyst. The method according to the invention is characterized by what is stated in independent claim 1. The required voltage is significantly lower than in electrolysis of water, i.e. approximately 0.2 to 0.3 V. Owing to this, the method according to the invention allows hydrogen to be produced with less energy. The invention is also characterized in that production of hydrogen can be implemented by way of self-sufficient energy supply because the electric energy generated of the produced hydrogen with a fuel cell is greater than the electric energy consumed for producing hydrogen in electrolysis.

The invention also relates to an electrolytic cell unit in which hydrogen is produced enzyme-catalytically from an aqueous solution of an alcohol. The electrolytic cell unit is characterized by what is stated in the independent claim.

According to the invention, hydrogen produced in an electrolytic cell unit can be further conveyed to a PEM type fuel cell in which the chemical energy of the hydrogen fuel is converted into electric energy. The electric energy required for electrolysis is obtained as part of the electric energy generated in this way and the rest may be further used to energize a device requiring electric power.

Thus, the invention also relates to a system comprising an electrolytic cell unit according to the invention for producing hydrogen, a PEM fuel cell using hydrogen and intended for generating electricity, and an electronic current control unit by means of which the hydrogen production of the electrolytic unit can be controlled to correspond to the consumption. The system according to the invention is applicable to be used as a power source particularly for portable electronic devices, such as portable computers, mobile phones, iPod devices, cameras etc. with a required power of 0.1 to 20 W. The invention enables a larger energy store to be carried with devices, compared with the use of accumulators and batteries.

The method and system according to the invention enable, with low manufacturing costs, production of an environmentally friendly, efficient and small-sized energy source which the end user will find easy to use and which can be made disposable and be destroyed in a safe manner without loading the environment. Compared with accumulators and batteries, the energy density may be multiplied because primary energy is stored in a chemical form in an organic fuel.

Compared with known reforming methods with organic substances where hydrogen is produced catalytically by means of heat and pressure, advantages of the method and system according to the invention include the fact that lower temperatures, such as room temperature, may be used, whereas metal-catalyzed reforming methods usually require high temperatures. An advantage is also the easy controllability of hydrogen production, 0 to 100% of the capacity simply by changing the electrode voltage/current, whereby hydrogen can be produced momentarily when required and needs not be stored as hydrogen. Chemical reforming methods usually have poor controllability of the hydrogen production reaction.

By means of the gas fuel cell system according to the invention, the same electric power is generated in a significantly smaller volume than in a corresponding liquid fuel cell using the same primary fuel. This is due to the power density of liquid fuel cells being lower than that of gas fuel cells. The system according to the invention also results in higher energy density than with a corresponding system in which the electrolysis of a fuel, such as methanol, is catalyzed by means of a noble metal, because in the case of a noble metal, the fuel content cannot be raised very high due to the poisoning tendency of the catalyst. The invention allows chemically bound hydrogen to be used as the primary fuel, which has a great logistic significance. With present methods, hydrogen must often be stored for logistic use in metal hydride containers, in which the energy density remains low.

### BRIEF DESCRIPTION OF THE DRAWINGS

Figure 1 shows an embodiment of an electrolytic cell unit according to the invention, and its enzyme-catalytic operating principle.
Figure 2 shows an embodiment of a system according to the invention, comprising an electrolytic cell unit according to the invention, a PEM-type fuel cell using fuel produced therein, and an electronic current control unit.

### DETAILED DESCRIPTION OF THE INVENTION

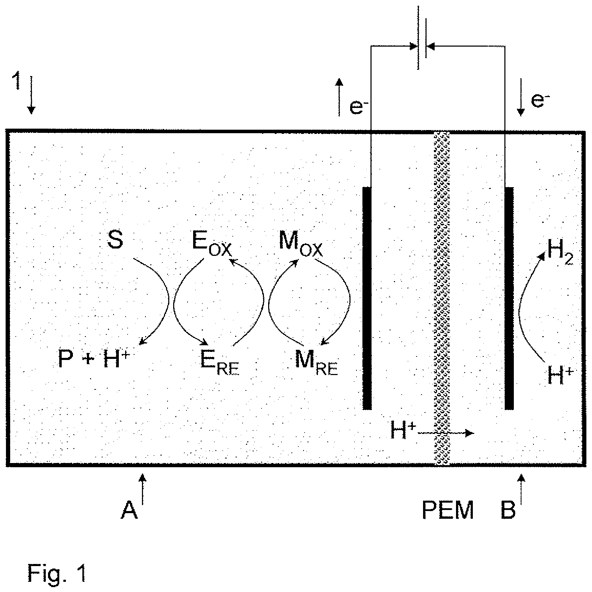

A method according to the invention for producing hydrogen and an embodiment of an electrolytic cell unit 1 according to the invention are illustrated in Figure 1. The unit comprises an anode-containing anode compartment (A) and a cathode-containing cathode compartment (B) as well as a proton exchange membrane (PEM) between them. The anode compartment contains an aqueous solution of an alcohol (substrate) as the primary hydrogen source, enzyme catalyst, buffer solution for controlling pH if required, and mediator as an intermediate oxidant for transferring electrons to the anode. The cathode compartment contains water and a suitable metal or enzyme catalyst which catalyzes the reduction reaction of protons into hydrogen. The electrolysis of a substrate is started by switching on the voltage between the electrodes in accordance with Figure 1, whereby the substrate is oxidized in the anode compartment. The electrons are transferred to the anode by means of a mediator in such a way that the mediator first oxidizes the enzyme, after which it oxidizes itself at the anode, transferring the electrons released in the decomposition reaction of the substrate to the anode. Symbol "S" in Figure 1 means a substrate and symbol "P" a product. From the anode, the electrons are further transferred to the cathode, generating an electric current.

Particularly pure alcohols in the form of an aqueous mixture are suitable to be used as an organic substrate serving as the primary hydrogen source in the anode compartment (A). Particularly lower alcohols, such as methanol and ethanol, are suitable. The alcohol content in the mixture is appropriately as high as possible, i.e. at least 30% by volume. In an embodiment of the invention, the alcohol content is 30 to 40% by volume. Due to the high energy content, a particular embodiment of the invention uses an aqueous solution of 30% by volume of methanol.

Oxidation of the aqueous solution of an alcohol in the anode compartment of the electrolytic cell is catalyzed in accordance with the invention by means of an enzyme. A particular characterizing feature of the enzymes used is that they are enzymes functioning without an NADH cofactor (reduced nicotinamide adenine dinucleotide). It is also characteristic of the enzymes that they maintain high activity for a long time and remain active in high alcohol and sugar contents (40 to 45%).

The enzyme used is a PQQ (pyrroloquinoline quinone) dehydrogenase. In the case of an aqueous solution of methanol, a methanol dehydrogenase (MDH) is used. Finding a suitable enzyme in the case of each particular substrate belongs to the expertise of those skilled in the art. The amount of enzyme in a mixture of an aqueous solution of an alcohol is optimized according to the required hydrogen effect and the manufacturing costs.

The presence of a mediator in an electrolytic cell according to the invention is optional. Figure 1 shows an embodiment in which the anode compartment comprises mediator. The task of the mediator is to make the transfer of electrons which are formed in the anode compartment of the electrolytic cell according to the invention to the anode more efficient. Which mediator substance is selected depends of the enzyme used. If the substrate is methanol, for instance, and the catalyst is MDH (methanol dehydrogenase) enzyme decomposing it, the mediator may be N,N,N',N' tetramethylphenylenediamine (TMPD), for instance. Mediators are substances generally known and commercially available. They are not consumed in the reaction but change from one oxidation stage to another when transferring electrons. Selecting a suitable mediator belongs to the general expertise of those skilled in the art.

The protons are transferred from the anode compartment through a PEM membrane to the cathode compartment (B) and are reduced into hydrogen. The membrane in the electrolytic cell according to the invention may be any conventional proton exchange membrane with low permeability with respect to the organic substrate and mediator and with good proton conductivity, such as a Nafion^{®} membrane (manufactured by DuPont).

The cathode compartment (B) comprises not only a cathode but also water and an appropriate metal or enzyme catalyst that catalyzes the reduction reaction of the protons into hydrogen. The metal catalyst may be nickel, for example. Noble metals are also suitable. Enzyme catalysts may be hydrogenases, which include for instance EC 1.12.1.2 hydrogen dehydrogenase, EC 1.12.1.3 hydrogen dehydrogenase, EC 1.12.2.1 cytochrome-c3-hydrogenase and EC 1.12.7.2 ferredoxin hydrogenase.

The method according to the invention for electrolyzing an aqueous solution of an alcohol is characterized by the reaction not being completed into hydrogen and carbon dioxide when one enzyme is used, as generally happens when a noble metal catalyst is used. When methanol is used as the fuel, the reaction product in the electrolysis of methanol, catalyzed by an MDH enzyme, is formic acid. The reaction product is thus a liquid, which is more easily kept inside the electrolytic cell unit and removed as waste than gaseous carbon dioxide. In contrast, a reaction catalyzed by platinum is completed. Although hydrogen production may be considered quantitatively more efficient with a noble metal than enzymatically, this drawback is remedied by the higher methanol content enabled by the enzyme-catalyzed reaction, i.e. by a higher energy content fitting in the same volume. When a noble metal catalyst is used, the alcohol content is typically restricted to less than 5% because the carbon monoxide generated in higher contents easily poisons the catalyst.

The total reaction of the electrolytic cell unit according to the invention and the oxidation-reduction reactions taking place in the anode and cathode compartments may be presented as follows when the fuel is, by way of example, methanol:

Total reaction CH₃O + H₂O → CHOOH + 2 H₂

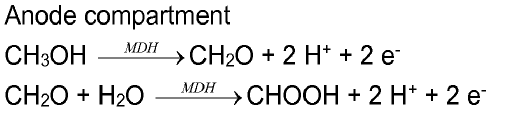

Cathode compartment 4 H⁺ + 4e⁻→2 H₂

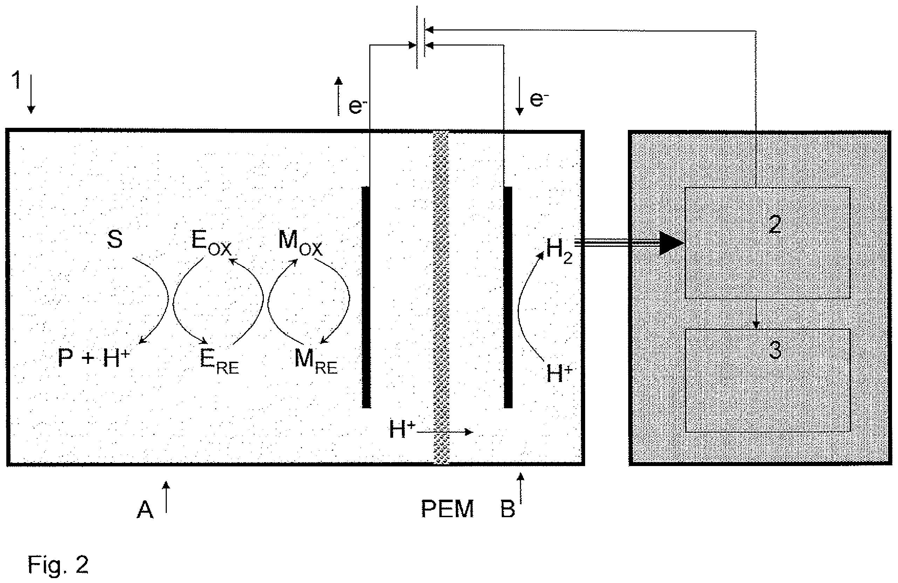

The electrolytic cell unit 1 according to the invention may be manufactured as a logistic product as a disposable cassette containing the aqueous solution of an alcohol used as the hydrogen source. Such a logistic exchangeable energy source unit is thus arrangeable in an electronic device which comprises, as a part of its structure, a PEM fuel cell 2 using hydrogen as the fuel, and an electronic current control unit 3 for appropriately controlling generation of electricity. This arrangement is shown in Figure 2.

In another embodiment of the invention, the logistic disposable product preferably in the form of a cassette may be an electrolytic cell unit 1 which is connected to a device formed of the PEM fuel cell 2 and the current control unit 3 for producing a controllable amount of hydrogen for the object of use.

Thus, Figure 2 shows an embodiment of the system according to the invention, comprising a hydrogen-producing electrolytic cell unit according to the invention, i.e. a "cassette" 1, and a PEM type fuel cell 2 for producing electric power, using the hydrogen produced as the fuel, as well as an electronic current control unit 3 providing a current for the electrolytic cell unit and intermediate storage of electricity of the application device, usable for buffering of momentary variations in the required power. The intermediate storage may be a small accumulator or a super capacitor. An aqueous solution of an alcohol (substrate) as the primary hydrogen source may be stored in a disposable cassette, for example, inside which the parts of the electrolytic device are made by using printing technology. The material may be, for instance, coated board or plastic. The aqueous solution of an alcohol is converted into hydrogen in the cassette by controlling the production according to the power consumption by means of electrode voltage. The hydrogen is conveyed to the power source of an electronic device, such as a mobile phone or a portable computer, in which it is converted into electricity by a small PEM fuel cell. The electrode voltage is taken from the current control unit 3 of the power source, which provides hydrogen production required by the electric power at the current moment.

The PEM fuel cell 2 and the electronic current control unit 3 may form a part of the structure of an application device, in which case the electrolytic cell unit 1 constitutes an exchangeable energy source in the form of a cassette for the PEM fuel cell. In another embodiment of the invention, the PEM fuel cell and the electronic current control unit may form an independent entity, the purpose of which is to energize the cassette to produce hydrogen for an object of use.

The electricity required for producing hydrogen is smaller in amount than the electricity generated of chemical energy. As becomes apparent from Figure 2, part of the electricity generated of hydrogen may, in accordance with a characterizing feature of the invention, be separated as electricity for producing hydrogen, and the rest may be utilized for practical applications. Thus, hydrogen production functions by way of self-sufficient energy supply. On the basis of experiments, it can be stated that on average, ¹/₃ of the electricity generated by a PEM fuel cell goes to electrolysis, ¹/₃ is converted into heat and ¹/₃ is obtained for utilization. The proportions vary depending on the type of the load condition in which the fuel cell is run.

It will be obvious to a person skilled in the art that as the technology advances, the basic idea of the invention may be implemented in a plurality of ways. The invention and its embodiments are thus not restricted to the above examples but may vary within the claims.

## Claims

1. A method for producing hydrogen, comprising decomposing an aqueous solution of an alcohol electrolytically in the presence of an enzyme catalyst to provide hydrogen, wherein the enzyme is a pyrroloquinoline quinone (PQQ) dehydrogenase.

2. The method according to claim 1, wherein the alcohol is methanol or ethanol.

3. The method according to claim 2, wherein an aqueous solution of methanol is used.

4. The method according to claim 3, wherein the methanol content is at least 30% by volume, particularly 30 to 40% by volume.

5. The method according to any one of preceding claims 1 to 4, wherein the enzyme is a dehydrogenase corresponding to the aqueous solution of an alcohol.

6. The method according to claim 5, wherein the enzyme is a methanol dehydrogenase.

7. An electrolytic cell unit (1) for producing hydrogen, comprising an anode compartment (A) provided with an anode, a cathode compartment (B) provided with a cathode, and a proton exchange membrane (PEM) between them, wherein the anode compartment comprises an aqueous solution of an alcohol, enzyme catalyst, which is a pyrroloquinoline quinone (PQQ) dehydrogenase, and, if required, a mediator for transferring electrons to the anode, and the cathode compartment comprises water and metal or enzyme catalyst.

8. The electrolytic cell unit according to claim 7, which is in the form of a logistic product.

9. The electrolytic cell unit according to claim 8, wherein the logistic product is in the form of an exchangeable cassette.

10. A system comprising an electrolytic cell unit (1) according to claim 7, a PEM fuel cell unit (2) and an electronic current control unit (3).

11. The system according to claim 10, wherein the electrolytic cell unit is connectable to the PEM fuel cell unit, whereby the electrolytic cell unit produces hydrogen to serve as fuel for the PEM fuel cell unit for generating electric power while the current control unit controls production of hydrogen to correspond to the load.

12. The system according to claim 10 or 11, wherein the electrolytic cell unit is in the form of a logistic product, particularly in the form of an exchangeable cassette, and connectable to a device formed of the PEM fuel cell unit and the current control unit.

13. The system according to claim 12, wherein the PEM fuel cell unit and the current control unit are arranged as a part of the structure of an application device.

14. The system according to claim 11 or 12, wherein the generated electric power is used for the electricity consumption of the electrolytic cell according to any one of claims 7 to 9.

15. The system according to any one of claims 11 to 13, wherein part of the generated electric power is used for the electricity consumption of an electrolytic cell according to any one of claims 7 to 9, the rest being used for the electricity consumption of the application device.

## Patentansprüche

1. Verfahren zur Wasserstoffherstellung, aufweisend elektrolytisches Zersetzen einer wässrigen Lösung eines Alkohols in der Anwesenheit eines Enzymkatalysators, um Wasserstoff bereitzustellen, wobei das Enzym eine Pyrrolquinolin-Quinon (PQQ) Dehydrogenase ist.

2. Verfahren nach Anspruch 1, wobei der Alkohol Methanol oder Ethanol ist.

3. Verfahren nach Anspruch 2, wobei eine wässrige Lösung von Methanol verwendet wird.

4. Verfahren nach Anspruch 3, wobei der Methanolgehalt zumindest 30 Volumen-%, insbesondere 30 bis 40 Volumen-% beträgt.

5. Verfahren nach einem der vorherigen Ansprüche 1 bis 4, wobei das Enzym entsprechend der wässrigen Lösung eines Alkohols eine Dehydrogenase ist.

6. Verfahren nach Anspruch 5, wobei das Enzym eine Methanoldehydrogenase ist.

7. Elektrolysezelleneinheit (1) zur Wasserstoffherstellung, aufweisend ein Anodenkompartiment (A), das mit einer Anode versehen ist, ein Kathodenkompartiment (B), das mit einer Kathode versehen ist, und eine Protonenaustauschmembran (PEM) zwischen diesen, wobei das Anodenkompartiment eine wässrige Lösung eines Alkohols, Enzymkatalysators, der eine Pyrroloquinolin-Quinon (PQQ) Dehydrogenase ist, und, sofern notwendig, einen Mediator zum Transferieren von Elektronen an die Anode aufweist, und wobei das Kathodenkompartiment Wasser und Metall oder einen Enzymkatalysator aufweist.

8. Elektrolysezelleneinheit nach Anspruch 7, welche in der Form eines Logistikprodukts vorliegt.

9. Elektrolysezelleneinheit nach Anspruch 8, wobei das Logistikprodukt in der Form einer austauschbaren Kassette vorliegt.

10. System, aufweisend eine Elektrolysezelleneinheit (1) nach Anspruch 7, eine PEM Brennstoffzelleneinheit (2) und eine elektronische Stromsteuereinheit (3).

11. System nach Anspruch 10, wobei die Elektrolysezelleneinheit mit der PEM Brennstoffzelleneinheit verbindbar ist, wobei die Elektrolysezelleneinheit Wasserstoff produziert, um als Brennstoff für die PEM Brennstoffzelle zum Generieren elektrischer Energie zu dienen, während die Stromsteuereinheit die Herstellung von Wasserstoff steuert, um der Last zu entsprechen.

12. System nach Anspruch 10 oder 11, wobei die Elektrolysezelleneinheit in der Form eines Logistikprodukts vorliegt, insbesondere in der Form einer austauschbaren Kassette, und mit einer Einrichtung, die aus der PEM Brennstoffzelle und der Stromsteuereinheit gebildet ist, verbindbar ist.

13. System nach Anspruch 12, wobei die PEM Brennstoffzelleneinheit und die Stromsteuereinheit als ein Teil der Struktur einer Applikationseinrichtung angeordnet sind.

14. System nach Anspruch 11 oder 12, wobei die erzeugte elektrische Energie für den Elektrizitätsverbrauch der Elektrolysezelle nach einem der Ansprüche 7 bis 9 verwendet wird.

15. System nach einem der Ansprüche 11 bis 13, wobei ein Teil der erzeugten elektrischen Energie für den Elektrizitätsverbrauch einer Elektrolysezelle nach einem der Ansprüche 7 bis 9 verwendet wird, wobei der Rest für den Elektrizitätsverbrauch der Applikationseinrichtung verwendet wird.

## Revendications

1. Procédé de production d'hydrogène, comprenant une décomposition d'une solution aqueuse d'un alcool par voie électrolytique en présence d'un catalyseur enzymatique pour fournir de l'hydrogène, dans lequel l'enzyme est une pyrroloquinoline quinone (PQQ) déshydrogénase.

2. Procédé selon la revendication 1, dans lequel l'alcool est du méthanol ou de l'éthanol.

3. Procédé selon la revendication 2, dans lequel une solution aqueuse de méthanol est utilisée.

4. Procédé selon la revendication 3, dans lequel la teneur en méthanol est au moins de 30 % en volume, en particulier de 30 à 40 % en volume.

5. Procédé selon l'une quelconque des revendications 1 à 4, dans lequel l'enzyme est une déshydrogénase correspondant à la solution aqueuse d'un alcool.

6. Procédé selon la revendication 5, dans lequel l'enzyme est une méthanol déshydrogénase.

7. Unité de cellule d'électrolyse (1) pour produire de l'hydrogène, comprenant un compartiment anodique (A) doté d'une anode, un compartiment cathodique (B) doté d'une cathode, et une membrane échangeuse de protons (PEM) entre eux, dans laquelle le compartiment anodique comprend une solution aqueuse d'un alcool, un catalyseur enzymatique, qui est une pyrroloquinoline quinone (PQQ) déshydrogénase, et, si nécessaire, un médiateur pour transférer des électrons vers l'anode, et le compartiment cathodique comprend de l'eau et un catalyseur métallique ou enzymatique.

8. Unité de cellule d'électrolyse selon la revendication 7, qui est sous la forme d'un produit logistique.

9. Unité de cellule d'électrolyse selon la revendication 8, dans laquelle le produit logistique est sous la forme d'une cassette échangeable.

10. Système comprenant une unité de cellule d'électrolyse (1) selon la revendication 7, une unité de cellule à combustible à PEM (2) et une unité de régulation de courant électronique (3).

11. Système selon la revendication 10, dans lequel l'unité de cellule d'électrolyse peut être connectée à l'unité de cellule à combustible à PEM, moyennant quoi l'unité de cellule d'électrolyse produit de l'hydrogène servant de combustible pour l'unité de cellule à combustible à PEM pour générer de l'énergie électrique tandis que l'unité de régulation de courant régule la production d'hydrogène pour correspondre à la charge.

12. Système selon la revendication 10 ou 11, dans lequel l'unité de cellule d'électrolyse est sous la forme d'un produit logistique, en particulier sous la forme d'une cassette échangeable, et peut être connectée à un dispositif formé de l'unité de cellule à combustible à PEM et de l'unité de régulation de courant.

13. Système selon la revendication 12, dans lequel l'unité de cellule à combustible à PEM et l'unité de régulation de courant sont agencées en tant que partie de la structure d'un dispositif d'application.

14. Système selon la revendication 11 ou 12, dans lequel l'énergie électrique générée est utilisée pour la consommation d'électricité de la cellule d'électrolyse selon l'une quelconque des revendications 7 à 9.

15. Système selon l'une quelconque des revendications 11 à 13, dans lequel une partie de l'énergie électrique générée est utilisée pour la consommation d'électricité d'une cellule d'électrolyse selon l'une quelconque des revendications 7 à 9, le reste étant utilisé pour la consommation d'électricité du dispositif d'application.
